(19) Europäisches Patentamt
European Patent Office
Office européen des brevets

(11) Veröffentlichungsnummer: **0 023 571**
**B1**

(12) **EUROPÄISCHE PATENTSCHRIFT**

(45) Veröffentlichungstag der Patentschrift: **12.01.83**

(51) Int. Cl.³: **C 08 G 63/62,** C 07 C 37/74, C 08 L 69/00

(21) Anmeldenummer: **80103697.1**

(22) Anmeldetag: **30.06.80**

(54) **Verfahren zur Herstellung von Polycarbonaten, die so erhältlichen Polycarbonate sowie ihre Verwendung.**

(30) Priorität: **13.07.79 DE 2928464**

(43) Veröffentlichungstag der Anmeldung: **11.02.81 Patentblatt 81/6**

(45) Bekanntmachung des Hinweises auf die Patenterteilung: **12.01.83 Patentblatt 83/2**

(84) Benannte Vertragsstaaten: **DE FR GB IT NL**

(56) Entgegenhaltungen:
**DD-A-57 133**
**DE-A-2 063 050**
**DE-A-2 329 585**
**DE-A-2 402 176**
**GB-A-1 052 618**

(73) Patentinhaber: **BAYER AG, Zentralbereich Patente, Marken und Lizenzen, D-5090 Leverkusen 1, Bayerwerk (DE)**

(72) Erfinder: **Friedhofen, Gerhard, Dr., Holzapfelweg 3, D-4150 Krefeld (DE)**
Erfinder: **Serini, Volker, Dr., Scheiblerstrasse 81, D-4150 Krefeld (DE)**
Erfinder: **Neumann, Rainer, Dr., Bodelschwinghstrasse 16, D-4150 Krefeld (DE)**
Erfinder: **Freitag, Dieter, Dr., Hasenheide 10, D-4150 Krefeld (DE)**
Erfinder: **Schwarz, Hans-Helmut, Dr., Ratherstrasse 90, D-4150 Krefeld (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 023 571 B1

ACTORUM AG

Verfahren zur Herstellung von Polycarbonaten, die so erhältlichen Polycarbonate sowie ihre Verwendung

Gegenstand der vorliegenden Erfindung ist ein Verfahren zur Herstellung von thermoplastischen, aromatischen, hochmolekularen Polycarbonaten aus speziellen Rohbisphenolen nach bekannten Verfahren, insbesondere nach dem Phasengrenzflächenverfahren, das dadurch gekennzeichnet ist, dass man Bisphenole einsetzt, die nach bekannten Verfahren hergestellt worden sind und unmittelbar danach von überschüssigen Phenolen und leichtsiedenden Substanzen in einem Verdampfer bei Temperaturen zwischen 100 und 300 °C, bevorzugt zwischen 150 und 250 °C, und bei Drücken zwischen 0,05 bis 1000 Millibar, bevorzugt zwischen 0,5 und 500 Millibar, abgetrennt worden sind und aus denen anschliessend mittels Desorption unter Verwendung von Inertgasen die restlichen Monophenole bis auf einen Gehalt von weniger als 2,5 Gew.-%, vorzugsweise von weniger als 0,5 Gew.-% an Monophenolen, bezogen auf Rückstand, entfernt worden sind.

Gegenstand der vorliegenden Erfindung sind ausserdem die nach dem erfindungsgemässen Verfahren erhältlichen hochmolekularen aromatischen Polycarbonate und ihre Verwendung als Formkörper, Überzüge, Fasern, Lacke und Folien, allein oder in Kombination mit anderen Stoffen.

Es ist bekannt, dass Bisphenole durch Kondensation von Phenolen mit Carbonylverbindungen, Biscarbinolen, Dienen oder Alkinen nach einer Vielzahl von Verfahren hergestellt werden können (siehe dazu beispielsweise US-A 2 191 831, US-A 2 845 464, DE-C 1 242 237, US-A 3 073 868, US-A 3 673 262, FR-A 1 374 477, US-A 3 394 089 und DE-A 2 422 532), wobei im Falle der Weiterverwendung für die Polycarbonatherstellung Ziel aller dieser Verfahren war, entweder durch spezielle Verfahrensführung oder durch aufwendige nachgeschaltete Reinigungsschritte Bisphenole hoher Reinheit zu erhalten. (Siehe dazu beispielsweise US-A 3 049 568, DE-C 1 186 874, DE-C 1 168 445, IT-A 650 774, DE-A 2 359 500, DE-A 2 364 164 und DE-A 2 537 027.

Der Grund dafür liegt darin, dass im allgemeinen nur Bisphenole hoher Reinheit zur Herstellung von hochmolekularen thermoplastischen Polycarbonaten einsetzbar waren (siehe beispielsweise DE-C 1 186 874 oder DT-A 2 537 027).

Auch gemäss nicht vorveröffentlichter deutscher Patentanmeldung P 28 11 182.2 setzt die Weitervewendung der Bisphenole für die Polycarbonatherstellung eine Kristallisationsstufe voraus, nämlich im für die Herstellung der Bisphenole verwendeten Austauscherharzbett.

Es war nun überraschend, dass nach bekannten Verfahren der Polycarbonatherstellung, insbesondere nach dem Verfahren der Phasengrenzflächenpolykondensation, ohne Einhaltung spezieller Reaktionsbedingungen aus Rohbisphenolen, vorzugsweise aus Rohbisphenolen, die mindestens 2 Alkylsubstituenten, insbesondere 4 Alkylsubstituenten besitzen, hochmolekulare, thermoplastische Polycarbonate der bekannt guten Qualität erhalten werden, wobei als besonderer Vorteil des Verfahrens die Tatsache zu bewerten ist, dass die Ausgangsprodukte für die Polycarbonatherstellung, nämlich die Rohbisphenole, auf besonders einfache Weise zur Verfügung stehen.

Eine einfache Möglichkeit zur Herstellung der erfindungsgemäss einsetzbaren Rohbisphenole besteht beispielsweise darin, dass man Phenole mit Carbonylverbindungen, Dienen, Alkinen oder Biscarbinolen, vorzugsweise mit Carbonylverbindungen, in Gegenwart saurer Katalysatoren im Molverhältnis der Phenole zu den anderen Reaktanten zwischen 2:1 und 40:1 bei Temperaturen unterhalb 120 °C, vorzugsweise unterhalb 80 °C umsetzt, überschüssiges Phenol und leichtsiedende Substanz in einem Verdampfer bei Temperaturen zwischen 100 °C und 300 °C, vorzugsweise bei Temperaturen zwischen 150 °C und 250 °C, und bei Drücken zwischen 0,05 und 1000 Millibar, vorzugsweise zwischen 0,5 und 500 Millibar abtrennt und anschliessend mittels Desorption im entsprechenden Temperaturbereich und im entsprechenden Druckbereich unter Verwendung von Inertgas, insbesondere von Stickstoff, die restlichen Monophenole bis auf einen Gehalt von weniger als 2,5 Gew.-%, vorzugsweise von weniger als 0,5 Gew.-% an Monophenolen, bezogen auf Rückstand, entfernt.

Einsetzbare Rohbisphenole im Sinne vorliegender Erfindung sind vorzugsweise Rohbisphenole, die 2 Alkylsubstituenten und insbesondere 4 Alkylsubstituenten haben und die noch andere phenolische Komponenten in untergeordneten Mengen enthalten.

Der Einsatz von Rohbisphenolen gemäss vorliegender Erfindung beinhaltet zumindest zwei Vorteile; zum einen ist er energiesparend, da die Rohbisphenole Sumpfprodukte sind, zum anderen können die abgetrennten Monophenole wieder in die Bisphenolherstellung zurückgeschleust werden.

Phenole für vorstehend erwähnte Rohbisphenolherstellung sind z.B. folgende der allgemeinen Formel

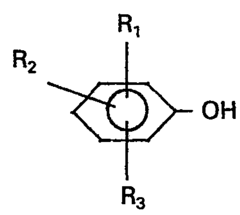

(1)

worin

$R_1$, $R_2$ und $R_3$ gleich oder verschieden sind und H, $CH_3$, $C_2H_5$ oder $C_3H_7$ bedeuten.

Geeignete Phenole sind beispielsweise Phenol, Kresole und 2,6-Dialkylphenole, insbesondere 2,6-Dimethylphenol.

Für vorstehend erwähnte Rohbisphenolherstellung geeignete Carbonylverbindungen sind solche, die für die Bisphenolherstellung in der Literatur beschrieben sind. Solche Carbonylverbindungen sind zum Beispiel folgende der allgemeinen Formel (2)

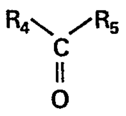 (2)

worin $R_4$ und $R_5$ gleich oder verschieden sind und H oder $C_nH_{2n+1}$ mit n=1 bis 15 bedeuten, oder der allgemeinen Formel (3)

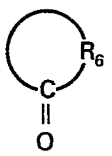 (3)

worin $R_6$ einen zweibindigen Rest $-CH_2-(CH_2)_m-CH_2-$ mit m=3 bis 13 bedeutet.

Bevorzugte Carbonylverbindungen sind Formaldehyd, Aceton, Butanon und Cyclohexanon.

Für vorstehend erwähnte einfache Möglichkeiten der Rohbisphenolherstellung geeignete Diene sind solche, die in der Literatur für die Herstellung von Bisphenolen beschrieben sind. Solche Diene sind zum Beispiel Alkyl- und Dialkylbutadiene, wie Methyl- und Dimethylbutadien.

Bevorzugte Diene sind Butadien, 2,3-Dimethylbutadien-1,3, 2-Methylbutadien-1,3.

Für vorstehend erwähnte einfache Möglichkeit der Rohbisphenolherstellung geeignete Alkine sind solche, die in der Literatur für die Herstellung von Bisphenolen beschrieben sind. Solche Alkine sind zum Beispiel folgende der allgemeinen Formel (4)

$$R_7-C\equiv C-R_8, \quad (4)$$

worin $R_7$ und $R_8$ gleich oder verschieden sind und H oder $C_nH_{2n+1}$ mit n=1 bis 5 bedeuten.

Bevorzugte Alkine sind Äthin, Propin, Butine.

Für vorstehend erwähnte einfache Möglichkeiten der Rohbisphenolherstellung geeignete Biscarbinole sind solche, die in der Literatur beschrieben sind, wie z.B.

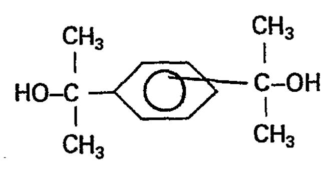

Bevorzugte Biscarbinole sind 1,4-Bis-2-(2hydroxypropyl)-benzol, 1,3-Bis-2-(2-hydroxypropyl)-benzol.

Für vorstehend erwähnte einfache Möglichkeit der Rohbisphenolherstellung geeignete saure Katalysatoren sind solche, die in der Literatur für die Herstellung von Bisphenolen beschrieben sind. Solche sauren Katalysatoren sind zum Beispiel Salzsäure, Schwefelsäure, Phosphorsäuren, Sulfonsäuren und saure organische Ionentauscher.

Leichtsiedende Substanzen im Sinne des erfindungsgemässen Verfahrens können zum Beispiel $H_2O$, Carbonylverbindungen, Diene, Alkine, Biscarbinole und saure Katalysatoren sein.

Als Verdampfer für das erfindungsgemässe Verfahren eignen sich beispielsweise Fallfilmverdampfer, Fallstromverdampfer, Dünnschichtverdampfer, Blasenverdampfer und Spiralverdampfer. Weitere technische Ausführungsformen sind in der Fachliteratur beschrieben (siehe dazu: «Ullmanns Encyclopädie der technischen Chemie, 3. Auflage, Verlag Urban und Schwarzenberg, München-Berlin, 1951, Band 1).

Bevorzugt erfolgt die Verdampfung kontinuierlich. Doch auch die diskontinuierliche Verdampfung ist möglich, wobei die Temperaturen und der Druck als Destillationsbedingungen konstant gehalten werden können oder im Sumpf kontinuierlich oder schrittweise verändert werden.

Als Inertgas für die Desorption gemäss dem erfindungsgemässen Verfahren kann grundsätzlich jedes unter den Reaktionsbedingungen inerte Gas oder jeder unter den Reaktionsbedingungen inerte Dampf eingesetzt werden, ausser Stickstoff beispielsweise auch Ar, He, Ne, $CO_2$, CO, $H_2$, $H_2O$-Dampf, Methan oder Halogenkohlenwasserstoffe.

Als Apparaturen für die Desorptionsstufe des erfindungsgemässen Verfahrens dienen beispielsweise Fallfilm-, Fallstrom- oder Dünnschichtverdampfer.

Bevorzugte Ausführungsform der Desorption ist eine Kolonne mit Böden oder Füllkörpern wie sie in der oben genannten Literatur beschrieben ist.

Die Anordnung der Desorptionsstufe kann unmittelbar an die Verdampferstufe für die Phenole anschliessen, gegebenenfalls kann ein Zwischenbehälter zwischen Verdampferstufe und Desorptionsstufe mit oder ohne Förderorgan eingesetzt werden.

Die Desorption kann im gleichen Temperaturbereich wie die Verdampfung erfolgen, ebenso kann der Druck bei der Desorption im gleichen Bereich wie bei der Verdampfung liegen. Im einzelnen müssen jedoch weder Druck noch Temperatur übereinstimmen, vielmehr können innerhalb der möglichen Bereiche von Temperatur und Druck jeweils unterschiedliche Einstellungen in der Verdampferstufe und in der Desorptionsstufe gewählt werden.

Die Menge an einzusetzendem Inertgas in der Desorptionsstufe beträgt etwa zwischen 0,05 und 50 $m^3$ pro kg zu desorbierender Phenole.

Üblicherweise wird die Desorption kontinuierlich durchgeführt, jedoch ist auch eine diskontinuierliche Fahrweise möglich. Temperatur und Druck können bei diskontinuierlicher Durchführung konstant gehalten werden. Eine stetige oder schrittweise Veränderung der Temperatur und des Druckes und der Inertgasmenge ist möglich.

Die erhaltenen Rohbisphenole lassen sich überraschend ohne besondere Reinigung, insbesondere ohne Kristallisation, Extraktion, Adsorption, Rektifikation, Sublimation oder Destillation, unmittelbar zur Herstellung von hochmolekularen

Polycarbonaten nach bekannten Verfahren einsetzen.

Geeignet sind beispielsweise Rohbisphenole, die mindestens 50 Gew.-%, bevorzugt 80 Gew.-% und besonders bevorzugt 90 Gew.-% eines der folgenden Bisphenole enthalten: α,α'-Bis-(4-hydroxyphenyl)-p-diisopropylbenzol, α,α'-Bis-(4-hydroxy-3,5-dimethyl-phenyl)-p-diisopropylbenzol, 2,2-Bis-(4-hydroxyphenyl)-propan, 1,1-Bis-(4-hydroxyphenyl)-cyclohexan, 2,2-Bis-(3-methyl-4-hydroxyphenyl)-propan, Bis-(3,5-dimethyl-4-hydroxyphenyl)-methan oder 2,2-Bis-3,5-dimethyl-4-hydroxyphenyl)-propan, Bis-(4-hydroxyphenyl)-sulfid und Bis-(4-hydroxyphenyl)-sulfon.

Die Rohbisphenole haben eine Reinheit von höchstens 99%, im allgemeinen sind sie nicht reiner als 97% und oft liegen sie in ihrer Reinheit unter 95%. Die Verunreinigungen sind im allgemeinen überwiegend mehrkernige phenolische Verbindungen unbekannter Struktur.

Neben den Rohbisphenolen können noch die für die Polycarbonatherstellung üblichen reinen Bisphenole bei dem erfindungsgemässen Polycarbonatherstellungsverfahren in beliebigen Mengen mitverwendet werden; derartige bevorzugte Bisphenole sind:

Bis-(4-hydroxyphenyl)-sulfon, Bis-(4-hydroxyphenyl)-sulfid, 2,2-Bis-(4-hydroxyphenyl)-propan, 1,1-Bis-(4-hydroxyphenyl)-cyclohexan, 2,2-Bis-(3-methyl-4-hydroxyphenyl)-propan, Bis-(3,5-dimethyl-4-hydroxyphenyl)-methan, 2,2-Bis-(3,5-dimethyl-4-hydroxyphenyl)-propan, 2,2-Bis-(3,5-dichlor-4-hydroxyphenyl)-propan, 2,2-Bis-(3,5-dibrom-4-hydroxyphenyl)-propan, α,α-Bis-(4-hydroxyphenyl)-p-diisopropylbenzol, α,α'-Bis-(4-hydroxy-3,5-dimethylphenyl)-p-diisopropylbenzol.

Besonderes bevorzugt sind 2,2-Bis-(4-hydroxyphenyl)-propan und 2,2-Bis-(3,5-dimethyl-4-hydroxyphenyl)-propan.

Die Herstellung von thermoplastischen, aromatischen Polycarbonaten ist im Prinzip bekannt.

So können sie beispielsweise nach dem Schmelzumesterungsverfahren aus Bisphenol und Diphenylcarbonat oder nach dem Lösungsverfahren aus Bisphenol und Phosgen mit Pyridin als Hilfsbase oder nach dem Zweiphasengrenzflächenverfahren ebenfalls aus Bisphenol und Phosgen synthetisiert werden, wie es z.B. in der Monographie H. Schnell, Chemistry and Physics of Polycarbonates, New York-London-Sidney, Interscience Publishers 1964, Polymer Reviews, Vol. 9, in DE-A 2 063 050, 2 063 052, 1 570 703, 2 211 956, 2 211 957, 2 248 817, 2 615 038 beschrieben ist.

Die Rohbisphenole können im allgemeinen nach mindestens einem oder oben genannten Verfahren zu hochmolekularen Polycarbonaten umgesetzt werden. So können zum Beispiel Rohbisphenole mit überwiegendem Anteil an 2,2-Bis-(3,5-dimethyl-4-hydroxyphenyl)-propan nach dem Zweiphasengrenzflächenverfahren zu hochmolekularen Polycarbonaten umgesetzt werden (vergleiche DE-A 2 063 050 und 2 063 052).

Beim Zweiphasengrenzflächenverfahren werden die Rohbisphenole, als Alkalisalze gelöst in der wässrigen Phase, in Gegenwart von organischen Lösungsmitteln für das entstehende Polycarbonat, wie z.B. chlorierte Aliphaten oder Aromaten, wie Methylenchlorid und Chlorbenzol, oder Mischungen solcher Lösungsmittel, mit Phosgen umgesetzt. Bei der Kondensation wird die wässrige Phase alkalisch gehalten, meist durch zusätzlichen Alkalizusatz. Als Katalysatoren für die Kondensation werden beispielsweise tertiäre aliphatische Amine, wie Triäthylamin, Tributylamin, N-Äthylpiperidin oder Onium-Verbindungen, insbesondere quaternäre Alkylammoniumverbindungen, wie Tetrabutylammoniumbromid, oder Mischungen davon eingesetzt. Zur Begrenzung der Kettenlänge werden Kettenbegrenzer wie Phenole, z.B. Phenol, Kresol, Dimethylphenol und p-tert.-Butylphenol in üblicher Weise verwendet. Durch Zusatz mehrfunktioneller Verbindungen können auch verzweigte Polycarbonate erhalten werden.

Es zeigte sich, dass die erfindungsgemässe Verwendung der Rohbisphenole für das Phasengrenzflächenverfahren von besonderem Vorteil ist, da ihre Löslichkeit in alkalisch-wässriger Phase besonders gut ist und darüber hinaus eine ausserordentlich geringe Gelierungstendenz der entstehenden Polycarbonate in chlorierten Lösungsmitteln gegeben ist.

Von Vorteil für das Schmelzumesterungsverfahren ist die gute Schmelzbarkeit der Rohbisphenole, ebenso die geringe Kristallisationstendenz der Polycarbonate. Diese ist auch ganz allgemein für die thermoplastische Verarbeitung von Bedeutung. Von Vorteil für das Verfahren in homogener Phase ist die besonders gute Löslichkeit der Rohbisphenole in organischen Lösungsmitteln.

Die nach dem erfindungsgemässen Verfahren erhältlichen Polycarbonate weisen mittlere Molekulargewichte $\overline{M}_w$ von mindestens 20 000, bevorzugt von mindestens 25 000 auf. Es sind mittlere Molekulargewichte bis über $\overline{M}_w = 200\,000$ erreichbar. Im allgemeinen werden für die Anwendung auf dem Thermoplastensektor jedoch mittlere Molekulargewichte unter $\overline{M}_w = 100\,000$, insbesondere unter 60 000 bevorzugt.

Die nach dem erfindungsgemässen Verfahren aus Rohbisphenolen hergestellten hochmolekularen, aromatischen Polycarbonate weisen überraschenderweise weitgehend die gleichen guten Eigenschaften wie die herkömmlichen Polycarbonate aus gereinigten Bisphenolen auf. Sie zeigen zusätzlich bei einer Reihe von Eigenschaften sogar beträchtliche Verbesserungen. So besitzen sie im allgemeinen eine ausserordentlich gute Löslichkeit in organischen Lösungsmitteln, insbesondere auch in Lacklösemitteln und in polymerisierbaren Monomeren, wie z.B. Styrol, Acrylnitril, Vinylchlorid, Methylmethacrylat und Mischungen davon, was für Pfropfreaktionen von Vorteil ist. Sie können somit als Basis für die Herstellung von Pfropfpolymeren dienen.

Sie zeigen ausserdem verbesserte rheologische Eigenschaften in der Schmelze, so höhere Fliessfähigkeit und verbesserte Strukturviskosität. Verbessert wird auch die kritische Breite, so z.B. bei

Polycarbonaten aus überwiegend 2,2-Bis-(4-hydroxyphenyl)-propan. Verbessert wird weiterhin die Vernetzbarkeit von Filmen, z.B. durch Elektronenstrahlen, die für verschiedene Anwendungen von Bedeutung ist. Auch ist die Flammfestmachung in vielen Fällen dadurch erleichtert, dass für die gleiche Flammwidrigkeit geringere Mengen an Flammfestmitteln (wie z.B.) cokondensiertes 2,2-Bis-(3,5-dibrom-4-hydroxyphenyl)-propan) benötigt werden. In vielen Fällen wird auch die Kriechstromfestigkeit deutlich erhöht.

Der Erhalt bekannter Verträglichkeiten von Polycarbonaten mit anderen Polymeren ist besonders überraschend, da bekannt ist, dass schon kleine Veränderungen in Polymeren oft bedeutende Veränderungen der Verträglichkeit und somit der Eigenschaften mit sich bringen. So ist beispielsweise die Verträglichkeit von Polycarbonaten auf Basis von Rohbisphenolen mit überwiegend 2,2-Bis-(3,5-dimethyl-4-hydroxyphenyl)-propan gegenüber Polycarbonaten auf Basis des entsprechenden reinen Bisphenols mit Polyvinylchlorid (vergl. DE-A 2 402 176), mit Polycarbonaten auf Basis von 2,2-Bis-(4-hydroxyphenyl)-propan (vergl. DE-A 2 248 818) und mit Styrolpolymerisaten (vergl. DE-A 2 329 585 und DE-A 2 329 646) praktisch nicht verändert. Infolgedessen werden auch hervorragende Polymerlegierungen mit den erfindungsgemässen Polycarbonaten erhalten.

Von Bedeutung ist ebenso der Erhalt der Hydrolysestabilität der Polycarbonate, insbesondere der von Polycarbonaten auf Basis von Rohbisphenolen mit überwiegend 2,2-Bis-(3,5-dimethyl-4-hydroxyphenyl)-propan (vergl. DE-A 2 063 050, DE-A 2 211 957).

Die erfindungsgemässen Polycarbonate lassen sich sehr gut zu Formkörpern, Überzügen, Fasern, Lacken und Folien verarbeiten. Mit Vorteil können auch bestimmte Legierungen mit anderen Polymeren, wie z.B. Polyvinylchlorid, Styrolpolymerisate und Polycarbonate aus gereinigten Bisphenolen eingesetzt werden. Die erfindungsgemässen Polycarbonate und ihre Polymerlegierungen lassen sich auch in Mischungen mit Füllstoffen, wie z.B. Mineralien, Holzmehl und Russ, Verstärkungsstoffen, wie z.B. Glasfasern, Asbest und Kohlefasern, Effektstoffen, Farbstoffen, Pigmenten, Stabilisatoren, z.B. zur Thermo-, Oxidations- und UV-Stabilisierung, Gleit- und Entformungsmitteln, flammfestmachenden Zusätzen, wie z.B. halogenierten aromatischen Verbindungen, Metalloxiden und Metallsalzen, und weiteren Zusatzstoffen gut verwenden. Sie sind insbesondere dort von Vorteil, wo gute elektrische Eigenschaften, hohe Wärmestandfestigkeit, hohe Zähigkeit und hohe Verseifungsstabilität gefordert werden. So können sie beispielsweise für hochwertige elektrische Bauteile, Elektroisolierfolien, Rohrleitungen für alkalische und saure Wässer, Gehäuse, Teile für den Automobilsektor und Haushaltsgeräte dienen.

Beispiel 1

A) Die Herstellung des Tetramethylbisphenols A wird kontinuierlich in einem Festbettreaktor durchgeführt. Das Reaktionsrohr ist 1000 mm lang und hat einen Durchmesser von 100 mm. Durch Mantelbeheizung wird es auf 65 °C thermostatisiert. Es ist mit 6 l wasserfreiem $H^+$-Ionenaustauscher gefüllt. Durch dieses Rohr werden 600 ml Reaktionslösung/1 Stunde gepumpt. Bei Eintritt in den Reaktor hat die Reaktionslösung folgende Zusammensetzung:

| | |
|---|---|
| Dimethylphenol | 97,6 Gew.-% |
| Aceton | 2,3 Gew.-% |
| β-Mercaptopropionsäure | 0,1 Gew.-%. |

Am Reaktorausgang ergibt sich folgende Zusammensetzung:

| | |
|---|---|
| Dimethylphenol | 89,6 Gew.-% |
| Aceton | 0,4 Gew.-% |
| $H_2O$ | 0,5 Gew.-% |
| Tetramethylbisphenol-A | 9,1 Gew.-% |
| β-Mercaptopropions | 0,1 Gew.-% |
| unbekannte Komponenten | 0,2 Gew.-% |

Die Reaktionslösung wird in einem ölbeheizten Schlangenverdampfer mit 3,5 m Rohrlänge und 10 mm Rohrdurchmesser bei einer Temperatur von 190 °C bei 27 mbar kontinuierlich durchgesetzt. 2,6-Dimethylphenol wird über einen Zyklon, der mit einer kurzen Füllkörperkolonne versehen ist und in einem Kühler, der bei 50 °C betrieben wird, kondensiert. Das 2,2-Bis-(3,5-dimethyl-4-hydroxyphenyl)-propan, das noch 1,5% 2,6-Dimethylphenol enthält, strömt aus dem Schlangenverdampfer in einen Dünnschichtverdampfer mit Rotor als Desorber, der mit Öl auf 185 °C geheizt ist und unter dem gleichen Druck wie der Schlangenverdampfer betrieben wird. In den Desorber wird von unten ein Stickstoffstrom von 6 l/h eingeleitet.

Das Rohbisphenol (180 g/h) wird in einer Vorlage aufgefangen. Es enthält unter 0,1% 2,6-Dimethylphenol, 4,9% unbekannte Komponenten und 95% Bis-(3,5-dimethyl-4-hydroxyphenyl)-propan. Die Farbzahl des Rohbisphenols beträgt 600 (Hazen Farbzahl).

Aus diesem Rohbisphenol lässt sich ein Polycarbonat mit ausgezeichneten Eigenschaften herstellen (s. Beispiel 2).

B) Herstellung von Polycarbonat aus Rohbisphenol des Bsp. 1, A)

In 2286 ml destilliertem Wasser und 614,4 ml 45 %iger Natronlauge wurden 568,8 g Rohbisphenol aus Bsp. 1,A und 5,6 g Phenol gelöst. Nach Zugabe von 1490 ml Methylenchlorid, 448 ml Chlorbenzol, 7,72 g Tetrabutylammoniumbromid und 5,70 ml Tri-n-butylamin wurden unter Rühren 336,4 g Phosgen gasförmig in 60 min in das Gemisch geleitet. Nach der Einleitung von 25 g Phosgen wurden noch 96 ml 45%iger Natronlauge zugesetzt. Nach Zugabe von 5,6 ml Triäthylamin wurde 30 min. gerührt. Die organische Phase wurde dann zur Aufarbeitung nach dem Ansäuern mit verdünnter $H_3PO_4$ und Verdünnen mit Methylenchlorid mit $H_2O$ elektrolytfrei gewaschen. Das durch Abdampfen des organischen Lösungsmittels erhältliche Polycarbonat hatte eine relative

Viskosität $\eta rel = 1,296$ (gemessen an 0,5 g Polycarbonat in 100 ml $CH_2Cl_2$-Lösung bei 25 °C und zeigte hervorragende Eigenschaften.

Beispiel 2

A) Auf analoge Weise wie in Beispiel 1, A wurde Bisphenol A hergestellt, wobei 2,6-Dimethylphenol durch Phenol ersetzt wurde. Das Rohbisphenol enthält 0,1% Phenol, 8,3% mindestens 7 unbekannter Komponenten und 91,6% Bisphenol A.

Aus diesem Rohbisphenol lässt sich ein Polycarbonat mit ausgezeichneten Eigenschaften herstellen (s. Bsp. 4).

B) Herstellung von Polycarbonat aus Rohbisphenol des Bsp. 2, A).

In 1347 ml destilliertem Wasser wurden 204 ml 45%ige Natronlauge gelöst und 228 g Rohbisphenol des Beispiel 2, A) zugesetzt. Als das Rohbisphenol nahezu gelöst war, wurden 1381 ml Methylenchlorid zugesetzt. Unter kräftiger Rührung wurde Phosgen eingeleitet, bis der Ansatz frei von Bisphenolat war. Dabei wurde durch Zugaben 45%iger Natronlauge der pH bei 13–14 gehalten.

Die Temperatur wurde zwischen 20 und 30 °C gehalten. Die Phosgeneinleitzeit betrug 15 min. Nach dem Phosgeneinleiten wurden 0,75 ml Triäthylamin zugesetzt und bei 25 °C noch 30 min lang gerührt. Zur Aufarbeitung wurde die organische Phase nach dem Ansäuern mit verdünnter Phosphorsäure noch mit Wasser elektrolytfrei gewaschen. Das durch Abdampfen des Methylenchlorids erhältliche Polycarbonat hatte eine relative Viskosität $\eta rel = 1,312$ (gemessen an 0,5 g Polycarbonat in 100 ml $CH_2Cl_2$-Lösung bei 25 °C) und zeigte hervorragende Eigenschaften.

**Patentansprüche**

1. Verfahren- zur Herstellung von thermoplastischen, hochmolekularen aromatischen Polycarbonaten aus Rohbisphenolen nach bekannten Verfahren, dadurch gekennzeichnet, dass man Bisphenole einsetzt, die nach bekannten Verfahren hergestellt worden sind und unmittelbar danach von überschüssigen Phenolen und leichtsiedenden Substanzen in einem Verdampfer bei Temperaturen zwischen 100 und 300 °C und bei Drucken zwischen 0,05 und 1000 Millibar abgetrennt worden und anschliessend mittels Desorption unter Verwendung von Inertgas die restlichen Monophenole bis auf einen Gehalt von weniger als 2,5 Gew.-% an Monophenolen, bezogen auf Rückstand, entfernt worden sind.

2. Verfahren gemäss Anspruch 1, dadurch gekennzeichnet, dass von überschüssigen Phenolen und leichtsiedenden Substanzen bei Temperaturen zwischen 150 und 250 °C, abgetrennt worden ist.

3. Verfahren gemäss Anspruch 1 und 2, dadurch gekennzeichnet, dass von überschüssigen Phenolen und leichtsiedenden Substanzen bei Drücken zwischen 0,5 und 500 Millibar abgetrennt worden ist.

4. Verfahren gemäss Anspruch 1 bis 3, dadurch gekennzeichnet, dass Rohbisphenole mit mindestens 2 Alkylsubstituenten eingesetzt werden.

5. Verfahren gemäss Anspruch 4, dadurch gekennzeichnet, dass Rohbisphenole mit 4 Alkylsubstituenten eingesetzt werden.

6. Thermoplastische aromatische Polycarbonate erhältlich gemäss Verfahren der Ansprüche 1 bis 5.

7. Polycarbonate gemäss Anspruch 6, dadurch gekennzeichnet, dass ihnen andere Polymere zugemischt sind.

8. Polycarbonate gemäss Anspruch 7, dadurch gekennzeichnet, dass ihnen Polyvinylchlorid, Polycarbonate auf Basis von 2,2-Bis-(4-hydroxyphenyl)-propan oder Styrolpolymerisate zugemischt sind.

9. Verwendung der Polycarbonate gemäss Anspruch 6 zur Herstellung von Formkörpern, Überzügen, Fasern, Lacken und Folien.

10. Verwendung der Polycarbonate gemäss Anspruch 6 zur Herstellung von Pfropfpolymeren.

**Revendications**

1. Procédé de production de polycarbonates aromatiques thermoplastiques de haut poids moléculaire à partir de bisphénols bruts par des procédés connus, caractérisé en ce qu'on utilise des bisphénols qui ont été obtenus par des procédés connus et qui ont été séparés immédiatement après de phénols en excès et de substances de bas point d'ébullition dans un évaporateur à des températures comprises entre 100 et 300 °C et à des pressions comprises entre 0,05 et 1000 millibars et les monophénols restants ont ensuite été éliminés par désorption en utilisant un gaz inerte jusqu'à une teneur de moins de 2,5% en poids de monophénols, par rapport au résidu.

2. Procédé suivant la revendication 1, caractérisé en ce que la séparation des phénols en excès et des substances de bas point d'ébullition a été effectuée à des températures comprises entre 150 et 250 °C.

3. Procédé suivant les revendications 1 et 2, caractérisé en ce que la séparation des phénols en excès et des substances de bas point d'ébullition a été effectuée à des pressions comprises entre 0,5 et 500 millibars.

4. Procédé suivant les revendications 1 à 3, caractérisé en ce qu'on utilise des bisphénols bruts portant au moins deux substituants alkyle.

5. Procédé suivant la revendication 4, caractérisé en ce qu'on utilise des bisphénols bruts portant quatre substituants alkyle.

6. Des polycarbonates aromatiques thermoplastiques pouvant être obtenus par le procédé suivant les revendications 1 à 5.

7. Polycarbonates suivant la revendication 6, caractérisé en ce que d'autres polymères leur sont adjoints.

8. Polycarbonates suivant la revendication 7, caractérisés en ce que du chlorure de polyvinyle, despolycarbonates à base de 2,2-bis-(4-hydroxyphényl)-propane ou des polymérisats de styrène leur sont adjoints.

9. Utilisation des polycarbonates suivant la revendication 6, pour la production d'articles

moulés, de revêtements, de fibres, de vernis et de feuilles.

10. Utilisation des polycarbonates suivant la revendication 6, pour la production de polymères greffés.

**Claims**

1. Process for the preparation of thermoplastic, high molecular weight, aromatic polycarbonates from crude bisphenols by known methods, characterised in that bisphenols are used which have been prepared according to known processes and immediately thereafter have been separated from excess phenols and low-boiling substances in an evaporator at temperatures between 100 and 300 °C and under pressures between 0.05 and 1000 millibars and then the content of residual monophenols has been reduced, by desorption using an inert gas, to below 2.5% by weight, based on the residue.

2. Process according to Claim 1, characterised in that the crude bisphenols have been separated from excess phenols and low-boiling substances at temperatures between 150 and 250 °C.

3. Process according to Claim 1 and 2, characterised in that the crude bisphenols have been separated from excess phenols and low-boiling substances under pressures between 0.5 and 500 millibars.

4. Process according to Claim 1 to 3, characterised in that crude bisphenols having at least 2 alkyl substituents are used.

5. Process according to Claim 4, characterised in that crude bisphenols having 4 alkyl substituents are used.

6. Thermoplastic aromatic polycarbonates obtainable according to a process of Claims 1 to 5.

7. Polycarbonates according to Claim 6, characterised in that other polymers have been mixed therewith.

8. Polycarbonates according to Claim 7, characterised in that polyvinyl chloride, polycarbonates based on 2,2-bis-(4-hydroxyphenyl)-propane or styrene polymers have been mixed therewith.

9. Use of the polycarbonates according to Claim 6 for the production of moulded articles, coatings, fibres, lacquers and films.

10. Use of the polycarbonates according to Claim 6 for the production of graft polymers.